# EUROPEAN PATENT SPECIFICATION

(11) **EP 2 583 690 B1**
(45) Date of publication and mention of the grant of the patent: **07.12.2016**
(21) Application number: 12189252.5
(22) Date of filing: 19.10.2012
(51) Int. Cl.: A61K 41/00, A61K 47/48

(54) **Liposomes including an elastin-like polypeptide conjugated to a hydrophobic group, a chemosensitizer and an anticancer agent, and the use thereof**
Liposom mit Konjugat aus elastinartigem Polypeptid und hydrophober Gruppe, Chemosensitizer und Antikrebsmittel und Verwendung davon
Liposome comprenant un conjugué de polypeptide de type élastine et d'un groupe hydrophobe, un agent de chimiosensibilisation et un agent anticancéreux et son utilisation

(30) Priority: 19.10.2011 KR 20110107055; 27.09.2012 KR 20120108269
(43) Date of publication of application: 24.04.2013
(73) Proprietor: Samsung Electronics Co., Ltd, Gyeonggi-do 443-742 (KR)
(72) Inventor: Park, Sun-min, Gyeonggi-do (KR); Kim, Hyun-ryoung, Gyeonggi-do (KR); Kim, Min-sang, Gyeonggi-do (KR); Park, Jae-chan, Gyeonggi-do (KR); Chae, Su-young, Gyeonggi-do (KR)
(74) Representative: Grünecker Patent- und Rechtsanwälte PartG mbB

(56) References cited:
- US-A- 5 720 976
- BIKRAM MALAVOSKLISH ET AL: "Thermo-responsive systems for controlled drug delivery.", EXPERT OPINION ON DRUG DELIVERY OCT 2008, vol. 5, no. 10, October 2008 (2008-10), pages 1077-1091, XP8159163, ISSN: 1742-5247
- CHILKOTI A ET AL: "Design of thermally responsive, recombinant polypeptide carriers for targeted drug delivery", ADVANCED DRUG DELIVERY REVIEWS 20021018 NL, vol. 54, no. 8, 18 October 2002 (2002-10-18), pages 1093-1111, XP2690721, ISSN: 0169-409X
- JIANCHEN WANG ET AL: "In vitro cytotoxicity of Stealth liposomes co-encapsulating doxorubicin and verapamil on doxorubicin-resistant tumor cells", BIOLOGICAL & PHARMACEUTICAL BULLETIN (OF JAPAN), PHARMACEUTICAL SOCIETY OF JAPAN, TOKYO, JP, vol. 28, no. 5, 1 May 2005 (2005-05-01), pages 822-828, XP002652275, ISSN: 0918-6158
- KYUNGA NA ET AL: "Elastin-like polypeptide modified liposomes for enhancing cellular uptake into tumor cells", COLLOIDS AND SURFACES. B, BIOINTERFACES, ELSEVIER, AMSTERDAM, NL, vol. 91, 27 October 2011 (2011-10-27), pages 130-136, XP028347797, ISSN: 0927-7765, DOI: 10.1016/J.COLSURFB.2011.10.051 [retrieved on 2011-11-02]
- WU X S ET AL: "Conjugation of phosphatidylethanolamine to poly(N-isopropylacrylamide) for potential use in liposomal drug delivery systems", POLYMER, ELSEVIER SCIENCE PUBLISHERS B.V, GB, vol. 33, no. 21, 1 January 1992 (1992-01-01), pages 4659-4662, XP024117511, ISSN: 0032-3861, DOI: 10.1016/0032-3861(92)90432-V [retrieved on 1992-01-01]

## Description

### BACKGROUND

### 1. Field

The present disclosure relates to liposomes including elastin-like polypeptides conjugated to moieties containing hydrophobic groups, chemosensitizers, and anticancer agents, pharmaceutical compositions including the liposomes. The disclosure also relates to such liposomes and pharmaceutical compositions for use in methods of delivering a chemosensitizer and an anticancer agent to a target site of a subject.

### 2. Description of the Related Art

Liposomes have at least one lipid layer membrane enclosing an aqueous internal compartment. Liposomes may be characterized by membrane type and by size. Small unilamellar vesicles (SUVs) have a single membrane and typically range between 20 and 50 nm in diameter. Large unilamellar vesicles (LUVs) may have a diameter of at least 50 nm. Oligolamellar large vesicles and multilamellar vesicles have multiple, usually concentric, membrane layers and may have a diameter of at least 100 nm. Liposomes with several nonconcentric membranes, i.e., several smaller vesicles contained within a larger vesicle, are termed multivesicular vesicles.

Liposomes are formulated to carry drugs or other active agents either contained within the aqueous interior space (water-soluble active agents or lipid-soluble active agents) or partitioned into the lipid bilayer (lipid-soluble active agents). In addition, a hydrophobic material such as cholesterol is contained in a micelle, and the micelle may be contained in the liposome interior space.

Active agents which have short half-lives in the bloodstream are particularly suited to delivery via liposomes. Many anti-neoplastic agents, for example, are known to have a short half-life in the bloodstream and thus, their parenteral use is not feasible. However, the use of liposomes for site-specific delivery of active agents via the bloodstream is severely limited by the rapid clearance of liposomes from the blood by cells of the reticuloendothelial system (RES).

Liposomes are not normally leaky unless a hole is formed in the liposome membrane, unless the membrane degrades or dissolves, or unless a temperature of the membrane increases to a phase transition temperature. The elevation of temperature at a target site in a subject (hyperthermia) may increase the temperature of the liposome to a phase transition temperature or higher and thus liposome contents may be released. This procedure may be used for the selective delivery of therapeutic agents. However, this technique is limited where the phase transition temperature of the liposome is significantly higher than the normal tissue temperature.

It is accordingly desirable to devise liposome formulations capable of efficiently delivering active agents.

### SUMMARY

Provided are liposomes including elastin-like polypeptides conjugated to moieties containing hydrophobic groups, chemosensitizers, and anticancer agents.

Provided are pharmaceutical compositions including the liposomes.

Provided are liposomes and pharmaceutical compositions for use in methods of delivering a chemosensitizer and an anticancer agent to a target site of a subject.

### BRIEF DESCRIPTION OF THE DRAWINGS

These and/or other aspects will become apparent and more readily appreciated from the following description of the embodiments, taken in conjunction with the accompanying drawings in which:
FIG. 1 is a graph showing the viability of NCI/ADR-RES cells when being cultured in the presence of doxorubicin, according to an embodiment;
FIG. 2 illustrates images showing the expression of drug-resistant genes in tumor cells, according to an embodiment;
FIG. 3 is a graph showing temperature-dependent release profiles of doxorubicin from a doxorubicin-containing liposome, according to an embodiment;
FIG. 4 is a graph showing storage time-dependent release of drugs at a storage temperature of 37°C, according to an embodiment;
FIG. 5 is a graph showing storage time-dependent release of drugs at a storage temperature of 42°C, according to an embodiment;
FIG. 6 is a graph showing cytotoxicities of free doxorubicin and a liposome co-encapsulating doxorubicin and verapamil against NCI/ADR-RES cells at a temperature of 37 °C, according to an embodiment;
FIGS. 7A and 7B are graphs showing cytotoxicities against free doxorubicin (7A) and a liposome co-encapsulating doxorubicin and verapamil (7B) at a temperature of 45 °C, according to embodiments; and
FIG. 8 is a graph showing an effect of the types of medicaments on cell viability, according to an embodiment.

### DETAILED DESCRIPTION

Reference will now be made in detail to embodiments, examples of which are illustrated in the accompanying drawings, wherein like reference numerals refer to like elements throughout. Accordingly, the embodiments are merely described below, by referring to the figures, to explain aspects of the present description. As used herein, the term "and/or" includes any and all combinations of one or more of the associated listed items.

According to an embodiment of the present invention, a liposome includes a lipid bilayer, elastin-like polypeptides (ELPs) conjugated to hydrophobic moieties, wherein the ELP comprises one or more repeating units of VPGXG, PGXGV, GXGVP, XGVPG, GVPGX, or combinations thereof, wherein V is valine, P is proline, G is glycine and X is any amino acid except proline, a chemosensitizer, and an anticancer agent, wherein the ELPs conjugated to hydrophobic moieties are packed in the lipid bilayer.

The term "lipid bilayer" as used herein indicates a membrane composed of two layers of lipid molecules. The lipid layer may have a similar thickness as that of a naturally existing bilayer, for example, a cell membrane, a nuclear membrane, or a virus envelope. For example, the thickness of the lipid bilayer may be 10 nm or less, for example, about 1 nm to about 9 nm, about 2nm to about 8 nm, about 2 nm to about 6 nm, about 2 nm to about 4 nm, or about 2.5 nm to about 3.5 nm. The lipid bilayer is a barrier that keeps such molecules where they are needed and prevents them from diffusing into areas where they should not be. Natural lipid bilayers are usually made mostly of phospholipids. A phospholipid has a hydrophilic head and two hydrophobic tails. When phospholipids are exposed to water, they arrange themselves into a two-layered sheet (a bilayer) with all of their tails pointing toward the center of the sheet. The center of this bilayer contains almost no water and also excludes molecules like sugars or salts that dissolve in water but not in oil. Phospholipids with certain head groups can alter the surface chemistry of a bilayer. Also, lipid tails may affect membrane properties, for instance by determining the phase of the bilayer. The bilayer can adopt a solid gel phase state at lower temperatures but undergo phase transition to a fluid state at higher temperatures. The packing of lipids within the bilayer also affects its mechanical properties, including its resistance to stretching and bending. Biological membranes typically include several types of lipids other than phospholipids. A particularly important example in animal cells is cholesterol, which helps strengthen the bilayer and decrease its permeability

A "lipid molecule" for constructing the lipid bilayer may be a molecule having a hydrophilic head and hydrophobic tails. The lipid molecule may have, for example, 14 to 50 carbon atoms. The lipid molecule may be phospholipid. The phospholipid may have 16 to 24 carbon atoms. Suitable phospholipids may be, for instance, phosphatidyl cholines, phosphatidyl glycerols, phosphaphatidyl inositols, phosphatidyl ethanolamines, and combinations thereof. In some embodiments, at least one phospholipid may have two acyl groups. Also, the phospholipid may have a phase transition temperature of about 10°C to about 70 °C, for example, about 20 °C to about 70 °C, about 30 °C to about 70 °C, about 40 °C to about 70 °C, about 50 °C to about 70 °C, about 60 °C to about 70 °C, about 10 °C to about 60 °C, about 10 °C to about 50 °C, about 10 °C to about 40 °C, about 10 °C to about 39 °C, about 30 °C to about 60 °C, about 30 °C to about 50 °C, about 30 °C to about 40 °C, about 30 °C to about 42 °C, about 30 °C to about 40 °C,about 30 °C to about 39 °C, about 35 °C to about 60 °C, about 35 °C to about 55 °C, about 35 °C to about 50 °C, about 35 °C to about 45 °C., about 35 °C to about 42 °C, about 35 °C to about 40 °C, about 35 °C to about 39 °C, about 38 °C to about 50 °C, about 38 °C to about 45 °C, about 38 °C to about 42 °C, about 38 °C to about 40 °C, or about 39 °C to about 45 °C. The acyl groups of the phospholipid may be saturated or unsaturated. The phospholipid may be a mixture of two or more of different phospholipid molecules. A lipid bilayer having various phase transition temperatures may be produced due to the mixture of two or more different phospholipid molecules.

A phospholipid molecule may have two acyl groups, for example, one selected from the group consisting of C12 saturated chain phospholipid (Tc=10 °C), a C14 saturated chain phospholipid (Tc=24 °C), a C16 saturated chain phospholipid (Tc=41 °C), a C18 saturated chain phospholipid (Tc=55 °C), a C20 saturated chain phospholipid (Tc=65 °C), a C22 saturated chain phospholipid (Tc=70 °C), and combinations thereof. Similarly, other common phospholipids that may be used include phosphatidyl glycerols, phosphatidyl inositols, phosphatidyl ethanolamines, sphingomyelins and gangliosides that, as with the phosphatidylcholines, have phase transition temperatures that vary in a similar fashion dependent on their acyl chain length. Tc refers to a phase transition temperature.

The C16 saturated chain phospholipid may be dipalmitoylphosphatidylcholine (DPPC). DPPC is a saturated chain (C16) phospholipid with a bilayer transition temperature of about 41.5 °C. An example of the C18 saturated chain phospholipid may be 1,2-distearoyl-sn-glycero-3-phosphocholine (DSPC). DSPC is a saturated chain (C18) phospholipid with a bilayer transition temperature of about 55.10 °C

Other membrane-forming lipid materials may be used which are not phospholipids. Exemplary materials which may form a solid-phase membrane include bola lipids or bacterial lipids. Additionally, block copolymers including a water-soluble polymer (e.g., polyethylene glycol) and a water-insoluble polymer (e.g., polypropylene oxide and polyethylethylene) may be employed.

As used herein, the "primary lipid" in a liposome bilayer is the main lipid component of liposome bilayer material. Thus, for example, in a liposome bilayer composed of 70 mole% phospholipid and 30 mole% cholesterol, the phospholipid is the primary lipid.

A lipid bilayer may have different phase behaviors that change with temperature. At a given temperature, a lipid bilayer may exist in either a liquid or a gel (solid) phase. Lipid molecules have a characteristic temperature at which they show transition from gel to liquid phase. In both phases, the lipid molecules are prevented from flip-flopping the bilayer, but in liquid phase bilayers, a given lipid will exchange locations with its neighbor. This random walk exchange allows lipids to diffuse and thus wander across the surface of the membrane. Unlike liquid phase bilayers, the lipids in a gel phase bilayer are locked in place.

The phase behavior of a lipid bilayer may be largely determined by the strength of the attractive forces of Van der Waals interactions between adjacent lipid molecules. Longer tailed lipid molecules have more area over which to interact, increasing the strength of this interaction and consequently decreasing the lipid molecule mobility. Thus, at a given temperature, a short-tailed lipid molecule will be more fluid than an otherwise identical long-tailed lipid molecule. Transition temperature may also be affected by the degree of unsaturation of the lipid molecule tails. An unsaturated double bond may produce a kink in the alkane chain, disrupting the lipid packing. This disruption creates extra free space within the bilayer which allows additional flexibility in the adjacent chains.

Most natural membranes are a complex mixture of different lipid molecules. If some of the components are liquid at a given temperature while others are in the gel phase, the two phases can coexist in spatially separated regions, rather like an iceberg floating in the ocean.

As used herein, the term "phase transition temperature" or "Tc" indicates the temperature at which a material changes from a solid phase to a liquid phase (also called a melting temperature) or from a liquid phase to a solid phase. The material includes lipid molecules, a lipid bilayer or liposome not including ELPs conjugated to hydrophobic moieties, or a lipid bilayer or liposome including ELPs conjugated to hydrophobic moieties. The phase transition temperature of a lipid membrane can be determined by differential scanning calorimertry (DSC), electron spin resonance (ESR) and the like. Differential scanning calorimetry may be performed with a model 4207 heatflow calorimeter from Hart Scientific. Samples may be prepared as described below, and the scan rate used may be 10°/h. Transition enthalpies may be estimated by using the peak integration software provided with the instrument. Transition entropies may be estimated from the transition enthalpies, assuming a first-order transition according to the expression Δt = Tₜ ΔSₜ, where ΔHₜ, ΔSₜ, and Tₜ, may be the transition enthalpy, entropy, and transition temperature, respectively. Samples for differential scanning calorimetry may be prepared by weighing about 3 to about 8 mg of the lipid into the DSC sample ampules and adding 0.5 ml of 10mM HEPES buffer, 1 mM EDTA (pH 7.4), with or without 1 M NaCl. The ampules may be then tightly sealed with screw caps and placed in the calorimeter. The reference ampule contained an equal volume of the buffer alone. Hydration of the liquid may be achieved by heating the samples above the chain-melting phase transition temperature (to 95 °C) in the calorimeter, incubating for 20 min, then cooling to 20 °C or lower and incubating for a further 20 min. After this hydration procedure, the samples may be subjected to two heating and two cooling scans, with nearly identical results for the repeated and repeated cooling scans.

The liposome includes ELPs conjugated to hydrophobic moieties, wherein the hydrophobic moieties may be packed in the lipid bilayer.

The hydrophobic moieties may constitute a lipid bilayer by being packed in the lipid bilayer. The hydrophobic moiety may be a molecule having a property of immobilizing the ELPs conjugated thereto to the lipid bilayer, for example, a hydrophobic property. The hydrophobic moiety may be the same as or different from a lipid molecule constituting the lipid bilayer.

The hydrophobic moiety may be provided by a molecule only containing a hydrophobic region, or by an amphipathic molecule containing both hydrophilic and hydrophobic regions. In the amphipathic molecules containing both hydrophilic and hydrophobic regions, the hydrophobic region may be arranged inwardly of the lipid bilayer, and the hydrophilic region may be arranged outwardly of the lipid bilayer and linked with ELPs. Here, "outwardly" of the lipid bilayer indicates a direction away from a center of the lipid bilayer, that is, inward of the liposome or outward of the liposome.

The hydrophobic moiety may be lipid molecules naturally existing in biomembranes, or lipid molecules that do not naturally exist in biomembranes and constitute the lipid bilayer.

The lipid molecules naturally existing in biomembranes may be selected from phospholipids or their derivatives, sterols or their derivatives, sphingolipids or their derivatives, and combinations thereof. The phospholipids or their derivatives may be selected from the group consisting of phosphatidyl cholines, phosphatidyl glycerols, phosphatidyl inositols, phosphatidyl ehtanolamines and combinations thereof. The sterols or their derivatives may be cholesterols or their derivatives, or squalenes or their derivatives. The sphingolipids may be sphingomyelins or their drivatives, or gangliosides or their derivatives. The phospholipids, sterols, or sphingolipids include intermediates or precursors produced during a synthesis process in vivo. For example, the hydrophobic moiety includes phosphoglycerides, sphingosines, ceramides, or cerebrosides.

The hydrophobic moiety may be a saturated or unsaturated hydrocarbon, a saturated or unsaturated acyl molecule, or a saturated or unsaturated alkoxy molecule.

A conjugation of a hydrophobic moiety and an ELP may be mediated via a non-cleavable linkage under physiological and pathological conditions or by a cleavable linkage. An example of the cleavable linkage may be a linkage mediated by a pH cleavable linker, a heat cleavable linker, a radiation cleavable linker, or a linker that is cleaved in aqueous solution.

The hydrophobic moiety may be conjugated to the ELP by binding with a nitrogen atom at the N-terminus of the ELP, or a carbonyl (-C(O)-) group at the C-terminus of the ELP. Alternatively, the hydrophobic moiety may be conjugated to the ELP by interaction with a functional group selected from an amino group, a carbonyl group, a hydroxyl group, a thiol group, or combination thereof. The hydrophobic moiety may be conjugated to the ELP by an amine bond or amide bond with a nitrogen atom of the ELP, or by an amide or ester bond with the carbonyl group at the C-terminus of the ELP.

The hydrophobic moiety may have 4 to 30 carbon atoms, for example, 14 to 24 carbon atoms or 16 to 24 carbon atoms. The hydrophobic moiety may be, for example, myristoyl (C14), palmitoyl (C16), stearoyl (C18), arachidonyl (C20), behenonyl (C22), or lignoceroyl (C24). The hydrophobic moiety may be packed in a lipid bilayer by a hydrophobic effect, and accordingly, the ELP conjugated to the hydrophobic moiety may be immobilized on the liposome.

As used herein the term "elastin-like polypeptides" refers to a class of amino acid polymers that undergo a conformation change dependent upon temperature. In an embodiment, the ELPs may be polymers exhibiting inverse phase transitioning behavior. Inverse phase transitioning behavior indicates that the ELPs are soluble in aqueous solutions below an inverse transition temperature (Tₜ), but the ELPs are insoluble as the temperature is raised higher than Tₜ. By increasing the temperature ELPs transition from elongated chains that are highly soluble into tightly folded aggregates with greatly reduced solubility. Such inverse phase transition may be induced by ELP structures having more β-turn structures and distorted β-structures as temperature increases. In some cases, ELPs may be defined based on the phase transitioning temperature. For example, in some cases, the phase transition may occur at a temperature from about 10 to about 70 °C.

When ELPs are linked to the components of a lipid bilayer, the inverse phase transitioning behavior may destroy the lipid bilayer due to shrinkage and self-assembly of the ELPs as temperature rises from a temperature lower than Tₜ of ELP to a higher temperature. Destroying the lipid bilayer may increase the permeability of the lipid bilayer. Thus, active agents contained in a liposome including the lipid bilayer may be released with a higher permeability from the liposome. However, one or more embodiments of the present invention are not limited to any particular mechanism.

The destruction of the lipid bilayer in a liposome due to the inverse phase transitioning behavior of ELP may differ according to lipid molecules of the lipid bilayer, or the phase transition temperature of the lipid bilayer. A lipid bilayer exists in a gel phase at the phase transition temperature or below and in a liquid (crystalline) phase at the phase transition temperature or above. When the lipid bilayer exists in a gel phase, destruction of the lipid bilayer may not occur or may be limited, though a structure of ELP changes to have a β-turn structure due to the inverse phase transitioning behavior. On the other hand, when the lipid bilayer exists in a liquid phase, the destruction of the lipid bilayer may be induced as a structure of ELP changes to have a β-turin structure due to the inverse phase transitioning behavior. In other words, when the lipid bilayer exists in a liquid phase rather than in a gel phase, the inverse phase transition induces destruction of the lipid bilayer more efficiently. Therefore, a releasing temperature of active agents contained in a liposome may be controlled by adjusting the phase transition temperature of a lipid bilayer of the liposome or the inverse phase transition temperature of ELP. For example, the phase transition temperature of a lipid bilayer or a liposome including ELPs may be from about 10 °C to about 70 °C, for example, about 39 °C to about 45 °C.

The ELP conjugated to a hydrophobic moiety may be conjugated to an end, rather than on a side chain, of the hydrophobic moiety. Also, as the ELP conjugated to a hydrophobic moiety is conjugated to an end, rather than on a side chain, the ELP may be conjugated to a hydrophobic moiety having one chain. For example, the hydrophobic moiety may be conjugated to the ELP by binding with a nitrogen atom at N-terminus or a carbonyl (-C(O)-) group at a C-terminus. The hydrophobic moiety may be conjugated to the ELP by an amine bond or an amide bond with the nitrogen atom of the ELP or by an amide or ester bond with the carbonyl group at the C-terminus of the ELP. Here, the hydrophobic moiety may have 4 to 30 carbon atoms, for example, 14 to 24 carbon atoms or 16 to 24 carbon atoms. In the ELP conjugated to a hydrophobic moiety, the ELP may be conjugated to a hydrophobic moiety having one chain.

A liposome including ELPs according to one or more embodiments may be used for efficiently releasing active agents contained in the liposome compared to a liposome not including ELPs but only a lipid bilayer. When simply a phase transition of lipid molecules of a lipid bilayer is used, the release of active agents in a liposome is induced by dispersion of the lipid molecules. Meanwhile, when a liposome including ELPs is used, a further release of active agents may be induced by the inverse phase transition behavior of ELP, in other words, further release of active agents may be induced by a destroyed lipid bilayer due to shrinkage and assembly of ELPs. Here, the active agents may be contained in an interior space of the liposome, in an interior of the lipid bilayer, or in both.

The ELP comprises one or more repeating units of VPGXG (SEQ NO: 1), PGXGV (SEQ NO: 2), GXGVP (SEQ NO: 3), XGVPG (SEQ NO: 4), GVPGX (SEQ NO: 5) and combinations thereof, where V is valine, P is proline, G is glycine, and X is any natural or non-natural amino acid except proline. Here, X in each repeating unit may be the same or different amino acid. The repeating units may be separated by one or more amino acids that do not remove a phase transition property of an obtained ELP, or an end portion may become the one or more amino acids or other linker moieties. A ratio of the repeating units verses the other amino acids or linker moieties may be about 0.1 to about 99.9% of the repeating units out of both the repeating units and the other amino acids. The selected repeating unit may be repeated twice or more, for example, 2 to 200 times.

In an embodiment, the ELP may be blocks where any one or more of VPGXG, PGXGV, GXGVP, XGVPG, GVPGX or a combination thereof is tandemly repeated, or the ELP may include blocks where VPGXG, PGXGV, GXGVP, XGVPG, GVPGX or combinations thereof is tandemly repeated. The ELP may include a repeating unit having one of the following formulae: [VPGXG]ₙ, [PGXGV]ₙ, [GXGVP]ₙ, [XGVPG]ₙ, [GVPGX]ₙ, or any combination of two or more repeating units identified herein. The ELP conjugated to a hydrophobic moiety may have the formula: C8-C24 fatty acy-[VPGXG]ₙ, [PGXGV]ₙ, [GXGVP]ₙ, [XGVPG]ₙ, [GVPGX]ₙ, or any combination of two or more repeating units identified herein. In the formula, n may be an integer of at least 1, such as of 1 to 200, 2 to 200, 2 to 100, 2 to 80, 2 to 60, 2 to 40, 2 to 12, 2 to 10, 2 to 8, 2 to 6, 4 to 100, 8 to 80, 10 to 60, 12 to 40, 20 to 40, 4 to 10, 4 to 8, or 4 to 6. For example, the ELP conjugated to a hydrophobic moiety may be stearoyl-VPGVG VPGVG VPGVG VPGVG VPGVG VPGVG-NH₂ (SEQ ID NO: 6, hereinafter, referred to as "SA-V6-NH₂").

As long as the inverse phase transition behavior is maintained, the ELP may be composed of VPGXG, PGXGV, GXGVP, XGVPG, GVPGX or a combination thereof and may include another portion in a molecule, for example a linker and blocking group. An N-terminus or C-terminus of the ELP may be linked with a hydrophobic moiety. Also, a hydrophobic moiety may be conjugated to an ELP by linking with a reactive group among a side chain of amino acid residue in the ELP. The reactive group may be an amino group, a hydroxyl group, a thiol group, or a carboxyl group. The other terminus not linked with a hydrophobic moiety may be blocked or unblocked. For example, when a hydrophobic moiety and an ELP are linked via the N-terminus of the ELP, a carboxyl group of the C-terminus of ELP may be blocked or unblocked. The blocking may be enabled by linking or interacting with a material that may be biocompatible, non-immunogenic, helpful in a specific delivery, or avoidable from biological degradation system. For example, the blocking may be enabled by an amide bond formed by binding a carboxyl group of a C-terminus of ELP and an amino group. The amino group may be an ammonia molecule, a primary amine, a secondary amine, or a tertiary amine. The primary, secondary, or tertiary amine may each have 1 to 10 carbon atoms, for example, 1 to 6 carbon atoms. X may be valine or alanine.

The repeating units may be each independently included in an ELP with one or more integer number of repetition. The number of repetitions may be each independently an integer of 2 to 200, 2 to 100, 2 to 80, 2 to 60, 2 to 40, 2 to 10, 2 to 12, 2 to 8, 2 to 6, 4 to 100, 8 to 80, 10 to 60, 12 to 40, 20 to 40, 4 to 10, 4 to 8, or 4 to 6.

In the liposome, a molar ratio of primary lipid molecules of the lipid bilayer: ELPs conjugated to a hydrophobic moiety may be appropriately selected according to a property of the selected lipid bilayer and a property of the ELPs conjugated to a hydrophobic moiety. For example, a molar ratio of primary lipid molecules: ELPs conjugated to a hydrophobic moiety may be about 50 to about 99.9: about 0.1 to about 50. For example, a molar ratio of primary lipid molecules (DPPC or mixtures of DPPC and DSPC): ELPs conjugated to a hydrophobic moiety (palmitoyl(VPGXG)ₙ or stearoyl(VPGXG)ₙ, where n is 2 to 12) may be about 50 to about 99.0: about 0.1 to about 50.

The term "chemosensitizer" as used herein is intended to include medicaments that make tumor cells more sensitive to the effects of chemotheraphy. The chemosensitizer may be a protein inhibitor that confers drug resistance. The chemosensitizer may be selected from the group consisting of multidrug resistance protein-1 (MDR1) inhibitors, MDR-2 inhibitors, multidrug resistance related protein-1 (MRP-1) inhibitors, breast cancer resistance protein (BCRP) inhibitors, and combinations thereof. The chemosensitizer may be selected from the group consisting of cyclosporin A, verapamil, bricodar, reversan, and combinations thereof.

The chemosensitizer may have an activity of a lipid bilayer stabilizing agent. For example, the chemosensitizer may be phenethylamine or a derivative thereof. Examples of phenethylamine or a derivative thereof include verapamil (i.e., (RS)-2-(3,4-dimethoxyphenyl)-5-{[2-(3,4-dimethoxyphenyl)ethyl]-(methyl)amino}-2-prop-2-ylpentanenitrile)) and derivatives thereof. Alternatively, the liposome may include a chemosensitizer but not include a lipid bilayer stabilizing agent. For example, the liposome may not include steroid or a derivative thereof, including cholesterol.

The anticancer agent may be an anthracycline-based anticancer agent. The anthracycline-based anticancer agent may be doxorubicin, daunorubicin, epirubicin, idarubicin, valrubicin, mitoxantrone, or a combination thereof.

The chemosensitizer may be contained in an interior space of the liposome, in an interior of the lipid bilayer, or in both. In addition, the chemosensitizer and the anticancer agent may be contained in the lipid bilayer and themselves have a lipid bilayer stabilizing activity and thus may be contained therein with or without a lipid bilayer stabilizing agent. The liposome may have a phase transition temperature from about 39 °C to about 45 °C. The liposome may be in a gel phase at room temperature.

The liposome may further include a lipid stabilizing agent. The lipid stabilizing agent may be a lipid having a phase transition temperature higher than a phase transition temperature of the lipid bilayer. The lipid bilayer stabilizing agent may be selected from the group consisting of steroids, sphingolipids or derivatives thereof, and combinations thereof. The lipid bilayer stabilizing agent may be steroid, a derivative thereof, or a combination thereof which has a property enabling incorporation into a lipid bilayer. As used herein, the term "steroid" indicates a type of organic compound including a core of gonane or a skeleton derived therefrom that contains a specific arrangement of four cycloalkane rings that are joined to each other, in other words, three cyclohexane rings designated as rings A, B, and C from left to right, and one cyclopentane ring (the D ring). Here, "a skeleton derived therefrom" includes an unsaturated bond inserted in the gonane skeleton. The steroids may vary depending on the functional groups attached to the four ring core and the oxidation state of the rings. For example, the steroids may include a hydrophilic functional group on the ring. For example, the steroids may have a hydroxyl group on the ring. The steroids may be sterols. The term "sterol" is a type of steroid which has the hydroxyl group at position C-3 and has a skeleton derived from cholestane. Here, the term "derived skeleton" includes an unsaturated bond inserted in the cholestane skeleton. The steroids include steroids found in plants, animals, and fungi. For example, all steroids may be made in cells either from lanosterol as in animals and fungi, or from cycloartenol as in plants. The sterols include cholesterols or their derivatives. Here, "derivative" means a derivate of cholesterol which maintains a property to be inserted in a lipid bilayer. The stabilizing agents may be selected from the group consisting of cholesterols, sitosterols, ergosterols, stigmasterols, 4,22-stigmastadien-3-ones, stigmasterol acetates, lanosterols, cycloartenols, and combinations thereof. The lipid bilayer stabilizing agent may be included in an effective amount so that the liposome is stably maintained at 37 °C but is disrupted at a certain temperature or higher, for example at 39 °C or higher. For example, the lipid bilayer stabilizing agent may be included in an effective amount so that the liposme is stably maintained at 37 °C for 30 minutes or longer but at least 50% of the liposome is disrupted at 39 °C or higher within 30 minutes.

A molar ratio of primary lipid molecules: the stabilizing agent, for example cholesterols, may be about 50 to about 99.9: about 0.1 to about 50. The ratio of the primary lipid molecules: the stabilizing agents may be about 50 to about 99.9: about 0.1 to about 50, for example about 50 to about 99.9: about 1 to about 50, about 50 to about 99.9: about 3 to about 50, about 50 to about 99.9: about 5 to about 50, about 50 to about 99.9: about 7 to about 50, about 50 to about 99.9: about 9 to about 50, about 50 to about 99.9: about 11 to about 50, about 50 to about 99.9: about 15 to about 50, about 50 to about 99.9: about 20 to about 50, about 50 to about 99.9: about 20 to about 35, about 50 to about 99.9: about 20 to about 30, about 50 to about 99.9: about 25 to about 30, about 50 to about 99.9: about 25 to about 50, about 50 to about 99.9: about 30 to about 50, about 50 to about 99.9: about 35 to about 50, about 50 to about 99.9: about 1 to about 35, about 50 to about 99.9: about 3 to about 30, about 50 to about 99.9: about 5 to about 25, about 50 to about 99.9: about 7 to about 20, or about 50 to about 99.9: about 9 to about 15.

Liposomes may not accumulate in leaky tumor tissue because of their relatively short half life in blood circulation due to their rapid uptake by macrophages of the liver and spleen (organs of the endothelial system or reticuloendothelial system (RES)). The term "leaky" or "leaky property" used in the specification refers to a property having an increased permeability of a material compared to a normal tissue or a cell. The target site may be tumor site, where the material permeability of blood vessels in tumors are increased due to leakiness of tumor vessels. Liposome preparation may be devised to avoid rapid RES uptake and thus increase circulation times. The lipid bilayer may contain, for example, lipids derivatives derivatized with hydrophilic polymers, for example phospholipids derivatives. The hydrophilic polymers may be selected from polyethylene glycol (PEG), polylactic acid, polyglycolic acid, copolymers of polylactic acid and polyglycolic acid, polyvinyl alcohols, polyvinyl pyrrolidone, oligosaccharide and mixtures thereof. The derivatives may be phospholipids of C4-C30, for example C16-C24, conjugated with PEG. The derivatives may be DPPC-PEG or DSPE-PEG. The PEG may have a weight average molecular weight of about 180 to about 50,000 Da

The liposomes may be unilamellar vesicles (SUV) or multivesiclufar vesicles. A diameter of the liposomes may be about 50 nm to about 500 nm, for example, about 50 nm to about 400 nm, about 50 nm to about 300 nm, about 50 nm to about 200 nm, about 100 nm to about 500 nm, about 100 nm to about 400 nm, about 100 nm to about 300 nm, or about 100 nm to about 200 nm.

In an embodiment, a liposome may include a phospholipid bilayer, ELPs conjugated to hydrophobic moieties, a chemosensitizer, and an anticancer agent, wherein the ELPs conjugated to hydrophobic moieties are packed in the phospholipid bilayer. The phospholipid bilayer includes phospholipid as a primary lipid molecule. The phospholipid may be DPPC, 1,2-distearoyl-sn-glycero-3-phosphocholine (DSPC), or a combination thereof. The phospholipid bilayer may contain phospholipid lipids derivatives derivatized with hydrophilic polymers, for example, a conjugate of 1,2-distearoyl-sn-glycero-3-phosphoethanolamine (DSPE) and PEG. The conjugate of DSPE and PEG may include 1,2-distearoyl-sn-glycero-3-phosphoethanolamine-N-[methoxy(polyethylene glycol)-2000] (ammonium salt). The phospholipid bilayer may include cholesterol.

In a particular embodiment, the liposome may include a phospholipid bilayer of DPPC, DSPC, or a combination thereof, stearoyl-VPGVG VPGVG VPGVG-NH₂, verapamil, and doxorubicin, wherein the stearoyl-VPGVG VPGVG VPGVG-NH₂ is packed in the phospholipid bilayer. A molar ratio of DPPC to DSPC in the phospholipid bilayer may be about 1: about 0 to about 0.5, for example, about 1: about 0.1 to about 0.5. The phospholipid bilayer may contain phospholipid lipids derivatives derivatized with hydrophilic polymers, for example, a conjugate of DSPE and PEG. The conjugate of DSPE and PEG may include 1,2-distearoyl-*sn*-glycero-3-phosphoethanolamine-N-[methoxy(polyethylene glycol)-2000] (ammonium salt). The phospholipid bilayer may include cholesterol.

The liposomes may have a phase transition temperature of about 10 °C to about 70 °C, for example, about 10 °C to about 60 °C, about 10 °C to about 55 °C, about 10 °C to about 45 °C, about 20 °C to about 60 °C, about 20 °C to about 55 °C, about 25 °C to about 45 °C, about 30 °C to about 45 °C, about 35 °C to about 45 °C, or about 39 °C to about 45 °C. The phase transition temperature may be adjusted by length of a carbon chain of primary lipid molecules, number of unsaturated bonds, mixtures of lipid molecules, and combinations thereof. For example, when DSPC with a phase transition temperature higher than that of DPPC is mixed with DPPC with a lower phase transition temperature, liposomes composed of the DPPC and DSPC mixture may have a higher phase transition temperature than that of liposomes only composed of DPPC. The liposomes may be in a gel phase at room temperature.

According to another embodiment of the present invention, a pharmaceutical composition for delivering a chemosensitizer and an anticancer agent to a target site of a subject includes a liposome and a pharmaceutically acceptable carrier or a diluent, wherein the liposome includes a lipid bilayer, ELPs conjugated to hydrophobic moieties, the chemosensitizer, and the anticancer agent, and the ELPs conjugated to hydrophobic moieties are packed in the lipid bilayer.

The pharmaceutically acceptable carrier or diluent may be well known in the art. The carrier or diluent may be selected from the group consisting of water, for example saline or sterile water, Ringer's solution, buffered saline, dextrose solution, maltodextose solution, glycerol, ethanol, and combinations thereof.

A detailed description of the liposome has already been described.

The liposome may be dispersed in an aqueous medium. The aqueous medium may include physiological saline or PBS.

According to another embodiment of the present invention, the liposomes and pharmaceutical compositions are for use in a method of delivering a chemosensitizer and an anticancer agent to a target site of a subject, wherein the liposome includes a lipid bilayer, ELPs conjugated to hydrophobic moieties, the chemosensitizer, and the anticancer agent, and the ELPs conjugated to hydrophobic moieties are packed in the lipid bilayer; and heating the target site of a subject to release the chemosensitizer and the anticancer agent from the liposome at the target site.

The method includes administering a liposome to a subject, wherein the liposome includes a lipid bilayer, ELPs conjugated to hydrophobic moieties, the chemosensitizer, and the anticancer agent, and the ELPs conjugated to hydrophobic moieties are packed in the lipid bilayer. A detailed description of the liposome has already been described.

The administration may be parenteral administration. The parenteral administration, for example, may be intravenous, intradermal, intramuscular, intracavity (abdominal cavity, joints, or eye), or direct injection. The direct injection may involve injecting directly into a diseased site such as a tumor site. The liposome may be administered intravenously and thereby brought to the target site such as a tumor site by blood flow. The target site may have a leaky property.

The method includes heating the target site of the subject to release the chemosensitizer and the anticancer agent from the liposomes at the target site. The heating may be due to a clinical procedure that induces hyperthermia or may be related to an intrinsically higher temperature of an inflamed body part compared to the rest of the body. The clinical procedure that induces hyperthermia may be performed by direct heat transfer, for example, a hot liquid medium in a tub, e.g., contacting a body in water, irradiating ultrasound, e.g., high intensity ultrasound focused at a target site, applying a magnetic field, e.g., an amplified magnetic field, applying microwave and/or radiofrequency. The target site may be a region where pathological symptoms exist, for example, a tumor site (i.e., a solid tumor), or where inflammation exists. The heating may be heating to a temperature of about 39 °C to about 45 °C.

The permeability of liposomes, according to an embodiment, may be adjusted by shrinking and self-assembling of ELPs conjugated to a hydrophobic moiety depending on a temperature. Therefore, the liposome may be used as a vehicle for effectively delivering a chemosensitizer and an anticancer agent to a target site of a subject.

According to a pharmaceutical composition for delivering a chemosensitizer and an anticancer agent to a target site of a subject, the chemosensitizer and the anticancer agent may be efficiently delivered to the target site of a subject.

According to a method of delivering a chemosensitizer and an anticancer agent to a target site of a subject, the chemosensitizer and the anticancer agent may be efficiently delivered to the target site of a subject.

One or more embodiments of the present invention will now be described more fully with reference to the following examples.

### Example 1: Preparation of liposomes and introduction of drugs thereinto

Liposomes were prepared using constituents and composition ratios shown in Table 1 below.

**<Table 1>**

| No. | Constituents and composition ratios | | | |
|---|---|---|---|---|
| | Phospholipid (mole) | DSPE-PEG^{*} (mole) | Cholesterol (mole) | ELP^{**} (mole) |
| 1 | 55 (DPPC) | 2 | 15 | 0.41 |
| 2 | 55 (DPPG) | 2 | 15 | 0.28 |
| 3 | 55 (DPPC) | 2 | 20 | 0.41 |
| 4 | 55 (DPPC) | 2 | 0 | 0.41 |
| 5 | 55 (DPPC:DSPC=1:3) | 2 | 0 | 0.41 |
| 6 | 55 (DPPC:DSPC=2:2) | 2 | 0 | 0.41 |
| 7 | 55 (DPPC:DSPC=3:1) | 2 | 0 | 0.41 |
| 8 | 55 (DPPC) | 2 | 10 | 0.41 |
| 9 | 55 (DPPC) | 2 | 5 | 0.41 |
| 10 | 55 (DPPC) | 2 | 15 | 0.83 |
| 11 | 55 (DPPC:DSPC=9:1) | 2 | 0 | 0.41 |
| 12 | 55 (DPPC:DSPC=8.5:1.5) | 2 | 0 | 0.41 |
| 13 | 55 (DPPC:DSPC=8:2) | 2 | 0 | 0.41 |

| | | | | |
|---|---|---|---|---|
| * 1,2-distearoyl-*sn*-glycero-3-phosphoethanolamine-N-[methoxy(polyethylene glycol)-2000] (ammonium salt) ** Stearoyl-VPGVG VPGVG VPGVG-NH₂ (SEQ ID NO: 7, hereinafter, referred to as SA-V3-NH₂) | | | | |

In particular, SA-V3-NH₂ was dissolved in ethanol, and DPPC (or DPPC/DSPC), DSPE-PEG and cholesterols were dissolved in chloroform. After mixing ethanol and chloroform solution in a round-bottom flask, a lipid thin layer was formed on the interior wall of the flask by evaporating the solvent at room temperature under reduced pressure using a rotary evaporator.

Next, the lipid thin layer was hydrated by adding a 150 mM ammonium sulfate solution to the flask at room temperature. The hydrated solution was subjected to vortexing and sonication treatment. Unilamella vesicle type liposomes were prepared by extruding the resulting solution using Avanti^{®} Mini-Extruder (Avanti Polar Lipids, Inc.) containing a polycarbonate film with pores having a size of 100 nm. A solvent of the prepared liposome solution was passed through a PD-10 (GE Healthcare) desalting column by flowing PBS therethrough so that PBS was exchanged with the solvent of the solution.

If necessary, two types of drugs, i.e., doxorubicin and verapamil, were loaded in the liposome. The loading process was performed using an ammonium sulfate gradient method (J. Control. Release 2009, 139, 73-80) or a pH-gradient method (Biochimica et Biophysica Acta 1985, 816, 294-302). In the loading process by the ammonium sulfate gradient method, a drug was added to the liposome solution formed of liposomes with 250 mM or 150 mM of ammonium sulfate inside and 25 mM of Tris-HCl buffer outside. The loading process was performed at a temperature of 37 °C for 60 minutes. In the loading process by the pH-gradient method, a drug was added to the liposome solution formed of liposomes with 300 mM of citrate buffer (pH 4.0) inside and 20 mM of HEPES buffer (150 mM NaCl, pH 7.4) outside. The loading process was performed at a temperature of 37 °C for 60 minutes.

The prepared liposome solution was passed through PD-10 (GE Healthcare) desalting column by flowing physiological saline therethrough to remove unentrapped drug. As a result, liposomes with the drug entrapped in the aqueous interior or a lipid bilayer thereof were prepared.

The sizes of the prepared liposomes were measured using a Zeta-sizer instrument (Malvern inst.). The liposomes had an average diameter of about 100 nm to about 1,000 nm. In addition, the sizes of the liposomes were adjusted by adjusting a composition ratio of constituents of the liposomes. For example, when the amount of ELPs was low, the sizes of the liposomes became smaller, and when the amount of cholesterol was large, the sizes of the liposomes became smaller.

Doxorubicin and verapamil entrapped in the liposomes were analyzed by high performance liquid chromatography (HPLC).

The liposomes co-encapsulting certain amounts of doxorubicin and verapamil were diluted and dissolved in dimethylsulfoxide (DMSO) and then introduced into a HPLC column. The analysis by HPLC was performed by eluting the drugs using KH₂PO₄/MeCN as an eluent and measuring absorbance thereof at 280 nm. As a result, intrinsic peaks of doxorubicin and verapamil were observed, which indicates that doxorubicin and verapamil were entrapped in the liposomes.

In addition, encapsulation amounts of the drugs were confirmed according to a loading method of the drugs. In the loading process, initial concentrations of doxorubicin and verapamil were 500 µg and 250 µg, respectively, based on 1 mL of the liposome solution. The amounts of the entrapped drugs by an ammonium sulfate gradient method and a pH-gradient method are shown in Table 2.

**<Table 2>**

| Loading method | Ammonium sulfate | gradient method | pH-gradient method |
|---|---|---|---|
| | 250mM ammonium sulfate (inside),25mM Tris HCl buffer (outside) | 150mM ammonium sulfate (inside),25mM Tris HCl buffer (outisde) | 300 mM citrate buffer (pH 4.0) (inside), 20 mM HEPES buffer (150 mM |
| | | | NaCl, pH 7.4) (outside) |
| Doxorubicin | 404 ug/mL | 36.5 ug/mL | 42 ug/mL |
| Doxorubicin/verap amil | 81.7 ug/mL/not confirmed | 40ug/mL, and 38.8ug/mL | Not confirmed |
| Verapamil | Not-confirmed | 1.4 ug/mL | Not-confirmed |

As shown in Table 2, when the ammonium sulfate (150 mM) gradient method was used, doxorubicin and verapamil were simultaneously entrapped in the liposomes.

In addition, particle diameters and polydispersity of the liposomes before and after the loading process were confirmed. Doxorubicin and verapamil were loaded on the prepared liposomes. The loading process was performed using the ammonium sulfate (150 mM) gradient method. Doxorubicin and verapamil were simultaneously added to the liposome solution at a mass ratio of 1:0.1 with respect to primary lipid and a mass ratio of 1:0.05 with respect to the primary lipid, respectively, and incubated at 37 °C for 1 hour.

**<Table 3>**

| Liposome No. | Before loading | | After loading | |
|---|---|---|---|---|
| | Average particle diameter (nm) | Polydispersity (pdi) | Average particle diameter (nm) | polydispersity (pdi) |
| 8 | 135 | 0.090 | 142.6 | 0.005 |
| 8 | 133.4 | 0.077 | 139.6 | 0.029 |
| 9 | 126.2 | 0.094 | 124.8 | 0.083 |
| 9 | 122 | 0.093 | 123.0 | 0.127 |

As shown in Table 3, even though one liposome encapsulated two drugs, there was little change in the particle diameter and polydispersity of the liposome. In other words, even though the hydrophobic drugs were introduced, aggregation was not observed.

### Example 2: Liposomes with doxorubicin and verapamil loaded thereon and effects thereof

Tumor cells having a resistance against doxorubicin, i.e., NCI/ADR-RES cells, were cultured in the presence of doxorubicin and the viability of the cells was evaluated. NCI/ADR-RES cells are tumor cells derived from OVCAR-8.

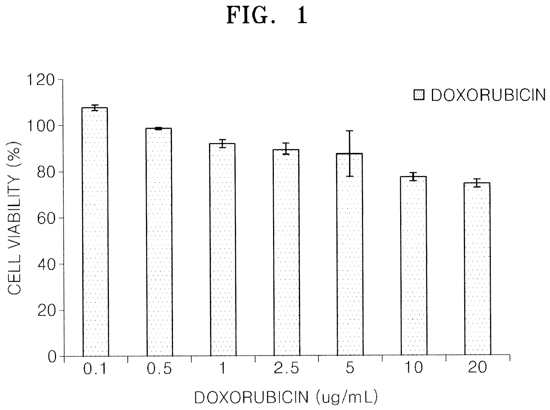

NCI/ADR-RES cells were incubated in minimum essential media (MEM) containing various concentrations of doxorubicin (0.1, 0.5, 1.0, 2.5, 5.0, 10 or 20 ug/mL), 10 volume% fetal bovine serum (FBS), and 1 wt% penicillin-streptomycin (PS) at 37 °C for 2 hours. After the MEM was replaced with fresh media, the cells were incubated at 37 °C for 2 days. The viability of the cells was evaluated by water soluble tetrazolium (WST) assay. FIG. 1 is a graph showing the viability of NCI/ADR-RES cells when being cultured in the presence of doxorubicin. As shown in FIG. 1, even though 20 ug/mL of doxorubicin was treated, more than about 70% of the NCI/ADR-RES cells were survived.

Next, it was confirmed whether drug-resistant genes were expressed in NCI/ADR-RES cells and other tumor cells. The drug-resistant genes used were MRP1, MDR1, and BCRP.

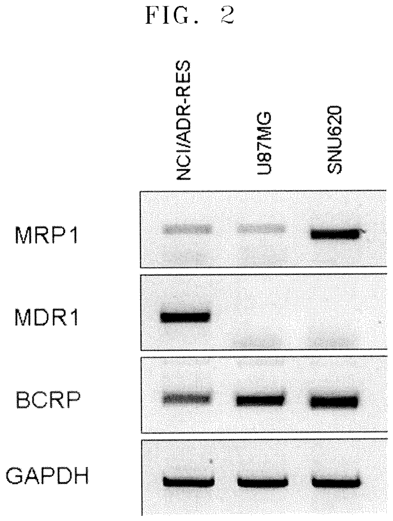

FIG. 2 illustrates images showing the expression of drug-resistant genes in tumor cells. As shown in FIG. 2, it was confirmed that at least two drug-resistant genes were expressed in tumor cells. FIG. 2 shows electrophoresis results of a product obtained by reverse transcription (RT)-polymerase chain reaction (PCR) using as a template a RNA sample derived from each cell.

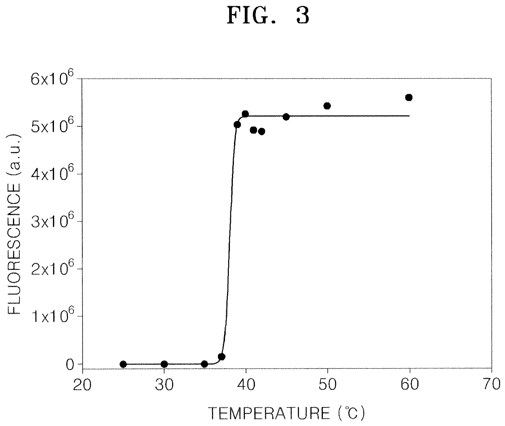

In addition, temperature-dependent release profiles of drugs from the liposomes containing the drugs were evaluated. FIG. 3 is a graph showing temperature-dependent release profiles of doxorubicin from a doxorubicin-containing liposome. The liposome used in FIG. 3 has a composition of Liposome No. 8 shown in FIG. 1. As shown in FIG. 3, the drug was not released until 37 °C since the liposome was stable, while the drug began to rapidly release at 39 °C.

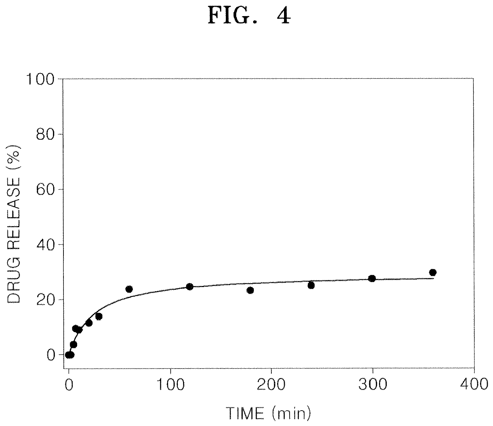

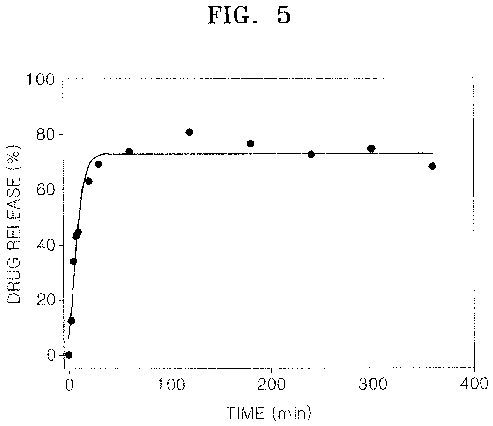

In addition, temperature sensitivity of the liposome co-encapsulating doxorubicin and verapamil was evaluated. 50% based on the weight of doxorubicin of verapamil, was contained in the liposome. FIG. 4 is a graph showing storage time-dependent release of drugs at a storage temperature of 37 °C. As shown in FIG. 4, the drugs were stably entrapped in the liposome at a temperature of 37 °C without release of the drugs. FIG. 5 is a graph showing storage time-dependent release of drugs at a storage temperature of 42 °C. As shown in FIG. 5, the drugs were rapidly released at a temperature of 42 °C within a short time. The release of drugs encapsulated in the liposome was measured using a plate reader. After incubation, the fluorescence intensity of the samples was measured at an excitation wavelength (λex)=485 nm and an emission wavelength (λem)=635 nm after suitable dilutions to determine the amount of doxorubicin released from the liposomes. The relative percent fluorescence intensity due to incubation at a particular temperature was calculated by comparison with the total release of entrapped material obtained after disruption of the liposome samples by adding 1% Triton X-100 (ethanol). The liposomes used in FIGS. 4 and 5 had a composition of Liposome No. 8 shown in Table 1.

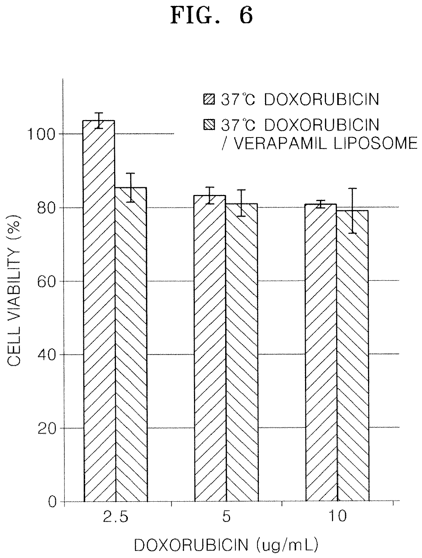

In addition, cytotoxicity of the liposome with doxorubicin and verapamil entrapped therein against NCI/ADR-RES cells was confirmed. FIG. 6 is a graph showing cytotoxicities of free doxorubicin and a liposome co-encapsulating doxorubicin and verapamil against NCI/ADR-RES cells at a temperature of 37 °C. 50% based on the weight of doxorubicin of verapamil, was contained in the liposome. As shown in FIG. 6, viability of the NCI/ADR-RES cells was lower in the liposome with doxorubicin and verapamil entrapped therein than in free doxorubicin. In this experiment, NCI/ADR-RES cells were incubated in each of a MEM containing free doxorubicin and a MEM containing the liposome with doxorubicin and verapamil entrapped therein, 10 volume% FBS, and 1 wt% PS at 37 °C for 48 hours. In particular, NCI/ADR-RES cells (5.0x10⁴ cells) were cultured in MEM containing 10% (v/v) FBS and 1% penicillin/streptomycin in a well of the 24-well plate for 48 hours. The cultured NCI/ADR-RES cells were treated with the liposomes containing doxorubicin and verapamil at a variety of concentrations, immediately placed in a thermoshaker, and then incubated at 37 °C for 10 minutes. Next, the NCI/ADR-RES cells were cultured at 37 °C for 2 hours and the MEM was then replaced with fresh media. Thereafter, the NCI/ADR-RES cells were cultured at 37 °C for 48 hours, and the viability of the NCI/ADR-RES cells was measured using CCK-8 assay kit (Dojindo) by WST (water soluble tetrazolium) assay. The liposome used in FIG. 6 had a composition of Liposome No. 8 shown in Table 1.

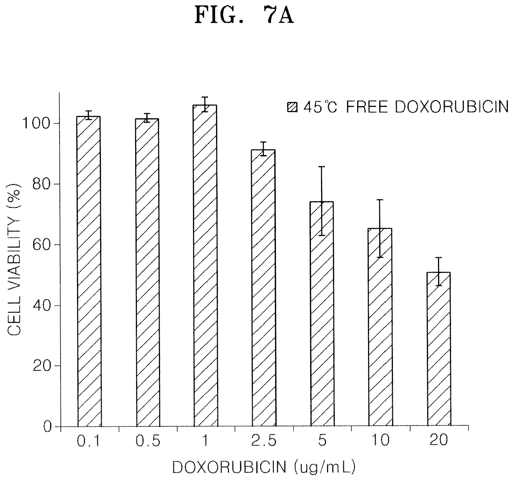

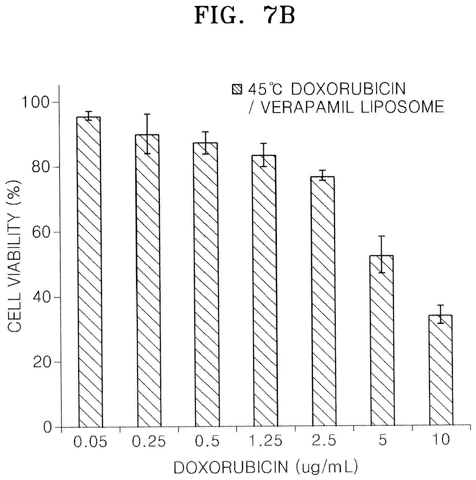

FIGS. 7A and 7B are graphs showing cytotoxicities against free doxorubicin (7A) and a liposome co-encapsulating doxorubicin and verapamil (7B) at a temperature of 45 °C. NCI/ADR-RES cells (5.0x10⁴ cells) were cultured in MEM containing 10% (v/v) FBS and 1% penicillin/streptomycin in a well of the 24-well plate for 48 hours. The cultured NCI/ADR-RES cells were treated with the liposomes containing doxorubicin and verapamil at a variety of concentrations, immediately placed in a thermoshaker, and then incubated at 45 °C for 10 minutes. 50% based on the weight of doxorubicin of verapamil, was contained in the liposome. Next, the NCI/ADR-RES cells were cultured at 37 °C for 2 hours and the MEM was then replaced with fresh media. Thereafter, the NCI/ADR-RES cells were cultured at 37 °C for 46 hours, and the viability of the NCI/ADR-RES cells was measured using CCK-8 assay kit (Dojindo) by WST assay. The liposome used in FIG. 7 had a composition of Liposome No. 8 shown in Table 1. As shown in FIG. 7A, when the NCI/ADR-RES cells were treated with a high concentration (20 ug/mL) of free doxorubicin, the cell viability was 50%. On the other hand, as shown in FIG. 7B, when the NCI/ADR-RES cells were treated with doxorubicin and verapamil, the cell viability was approximately 30% at a concentration of the liposome containing doxorubicin and verapamil of 10ug doxorubicin (+5ug verapamil)/mL. When the NCI/ADR-RES cells were treated with the same concentration (10ug/mL) of free doxorubicin, the cell viability was about 70%.

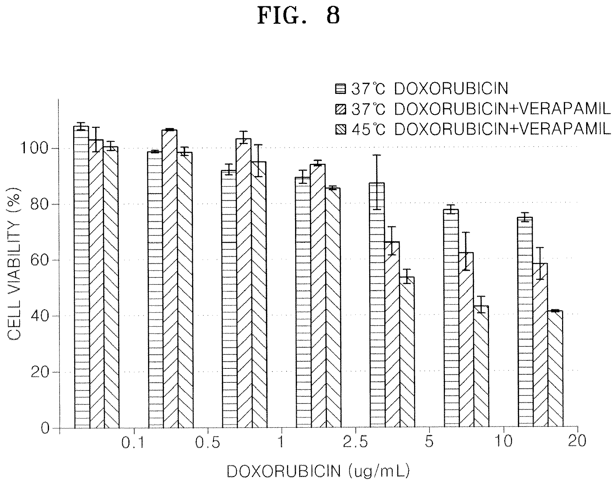

In addition, cytotoxicities of cells treated with free doxorubicin and cells simultaneously treated with free doxorubicin and free verapamil were confirmed. FIG. 8 is a graph showing an effect of the types of medicaments on cell viability. As shown in FIG. 8, the cell viability of a group simultaneously treated with doxorubicin and verapamil was lower than that of a group treated with doxorubicin alone. In this experiment, NCI/ADR-RES cells (5.0x10⁴ cells) were cultured in MEM containing 10% (v/v) FBS and 1% penicillin/streptomycin in a well of the 24-well plate for 48 hours. The cultured NCI/ADR-RES cells were treated with doxorubicin and verapamil at a variety of concentrations, immediately placed in a thermoshaker, and then incubated at 37°C or 45°C for 10 minutes. Next, the NCI/ADR-RES cells were cultured at 37 °C for 2 hours and the MEM was then replaced with fresh media. Thereafter, the NCI/ADR-RES cells were cultured at 37 °C for 46 hours, and the viability of the NCI/ADR-RES cells was measured using CCK-8 assay kit (Dojindo) by WST assay.

As a result of experiment, it was confirmed that the effect of an anticancer agent was improved by co-treating tumor cells with a chemosensitizer and an anticancer agent. In the case of *in vivo* treatment of tumor cells with doxorubicin and verapamil without being encapsulated in liposomes, the two drugs are unable to simultanelusly act on the tumor cells due to absorption site and amount of drugs, half-life thereof in the body, and the like, and thus, the effects of anticancer agents are improved at a low level. However, doxorubicin and verapamil are simultaneously released from liposomes co-encapsulating them to tumor sites by thermal stimulation and thus may be accumulated in the tumor sites, which results in an improved effect of the anticancer agent. The liposomes according to one or more embodiments may be used for the co-delivery of an anticancer agent and a chemosensitizer.
## Claims

1. A liposome comprising:
a lipid bilayer;
an elastin-like polypeptide (ELP) conjugated to a hydrophobic moiety, wherein the ELP comprises one or more repeating units of VPGXG, PGXGV, GXGVP, XGVPG, GVPGX, or combinations thereof, wherein V is valine, P is proline, G is glycine, and X is any amino acid except proline;
a chemosensitizer; and
an anticancer agent,
wherein the hydrophobic moiety and/or the ELP conjugated to the hydrophobic moiety is packed in the lipid bilayer.

2. The liposome of claim 1, wherein the chemosensitizer is contained in the interior space of the liposome, or in the lipid bilayer.

3. The liposome of claim 2, wherein the anticancer agent is contained in the interior space of the liposome.

4. The liposome of claim 1, wherein the chemosensitizer is selected from:
(a) the group consisting of multidrug resistance protein-1 (MDR1) inhibitors, MDR-2 inhibitors, multidrug resistance related protein-1 (MRP-1) inhibitors, breast cancer resistance protein (BCRP) inhibitors, and combinations thereof; and/or
(b) the group consisting of cyclosporin A, verapamil, bricodar, reversan, and combinations thereof.

5. The liposome of any of claims 1 to 4, wherein the anticancer agent is an anthracycline-based anticancer agent.

6. The liposome of any one of claims 1 to 5, further comprising a lipid bilayer stabilizing agent.

7. The liposome of claim 6, wherein the chemosensitizer and/or the anticancer agent has an activity of a lipid bilayer stabilizing agent.

8. The liposome of claim 6, wherein the lipid bilayer stabilizing agent comprises a steroid or a derivative thereof.

9. The liposome of any one of claims 1 to 8, wherein the repeating units are repeated 2 to 200 times.

10. The liposome of any one of claims 1 to 9, wherein the lipid bilayer comprises derivatives derivatized with hydrophilic polymers.

11. The liposome of any one of claim 10, wherein the lipid derivatized is a phospholipid derivative and/or the hydrophilic polymer is selected from polyethylene glycol (PEG), polylactic acid, polyglycolic acid, copolymers of polylactic acid and polyglycolic acid, polyvinyl alcohols, polyvinyl pyrrolidone, oligosaccharide and mixtures thereof.

12. The liposome of any one of claims 1 to 11, wherein the liposome has a transition temperature from 39°C to 45°C.

13. A pharmaceutical composition for delivering a chemosensitizer and an anticancer agent to a target site of a subject, the pharmaceutical composition comprising a liposome and a pharmaceutically acceptable carrier or a diluent, wherein the liposome is the liposome according to any one of claims 1 to 12.

14. The liposome of any one of claims 1 to 12 or the pharmaceutical composition of claim 13 for use in delivering a chemosensitizer and an anticancer agent to a target site of a subject.

## Patentansprüche

1. Liposom, umfassend:
eine Lipiddoppelschicht;
ein elastinartiges Polypeptid (ELP), das an einen hydrophoben Rest konjugiert ist, wobei das ELP eine oder mehrere Wiederholungseinheiten von VPGXG, PGXGV, GXGVP, XGVPG, GVPGX oder Kombinationen davon umfasst, wobei V Valin ist, P Prolin ist, G Glycin ist und X eine beliebige Aminosäure mit Ausnahme von Prolin ist;
einen Chemosensibilisator; und
ein Krebsmittel,
wobei der hydrophobe Rest und/oder das an den hydrophoben Rest konjugierte ELP in die Lipiddoppelschicht gepackt ist.

2. Liposom nach Anspruch 1, wobei der Chemosensibilisator in dem Innenraum des Liposoms oder in der Lipiddoppelschicht enthalten ist.

3. Liposom nach Anspruch 2, wobei das Krebsmittel in dem Innenraum des Liposoms enthalten ist.

4. Liposom nach Anspruch 1, wobei der Chemosensibilisator ausgewählt ist aus:
(a) der Gruppe bestehend aus Multidrug-Resistance-Protein-1 (MDR1)-Inhibitoren, MDR-2-Inhibitoren, Multidrug-Resistance-related-Protein-1 (MRP-1)-Inhibitoren, Brustkrebsresistenzprotein (BCRP)-Inhibitoren und Kombinationen davon; und/oder
(b) der Gruppe bestehend aus Cyclosporin A, Verapamil, Bricodar, Reversan, und Kombinationen davon.

5. Liposom nach einem der Ansprüche 1 bis 4, wobei das Krebsmittel ein Krebsmittel auf Anthracyclinbasis ist.

6. Liposom nach einem der Ansprüche 1 bis 5, außerdem umfassend ein Lipiddoppelschicht-Stabilisierungsmittel.

7. Liposom nach Anspruch 6, wobei der Chemosensibilisator und/oder das Krebsmittel eine Aktivität eines Lipiddoppelschicht-Stabilisierungsmittels aufweist.

8. Liposom nach Anspruch 6, wobei das Lipiddoppelschicht-Stabilisierungsmittel ein Steroid oder ein Derivat davon umfasst.

9. Liposom nach einem der Ansprüche 1 bis 8, wobei die Wiederholungseinheiten 2- bis 200-mal wiederholt werden.

10. Liposom nach einem der Ansprüche 1 bis 9, wobei die Lipiddoppelschicht Derivate umfasst, die mit hydrophilen Polymeren derivatisiert sind.

11. Liposom nach Anspruch 10, wobei das derivatisierte Lipid ein Phospholipidderivat ist und/oder das hydrophile Polymer ausgewählt ist aus Polyethylenglycol (PEG), Polymilchsäure, Polyglycolsäure, Copolymeren von Polymilchsäure und Polyglycolsäure, Polyvinylalkoholen, Polyvinylpyrrolidon, Oligosaccharid und Mischungen davon.

12. Liposom nach einem der Ansprüche 1 bis 11, wobei das Liposom eine Übergangstemperatur von 39 °C bis 45 °C aufweist.

13. Pharmazeutische Zusammensetzung zum Zuführen eines Chemosensibilisators und eines Krebsmittels zu einem Zielort eines Probanden, wobei die pharmazeutische Zusammensetzung ein Liposom und einen pharmazeutisch verträglichen Träger oder ein Verdünnungsmittel umfasst, wobei das Liposom das Liposom gemäß einem der Ansprüche 1 bis 12 ist.

14. Liposom nach einem der Ansprüche 1 bis 12 oder pharmazeutische Zusammensetzung nach Anspruch 13 zur Verwendung beim Zuführen eines Chemosensibilisators und eines Krebsmittels zu einem Zielort eines Probanden.

## Revendications

1. Liposome comprenant:
une bicouche lipidique ;
un polypeptide de type élastine (ELP) conjugué à un fragment hydrophobe, l'ELP comprenant un ou plusieurs motifs répétés parmi VPGXG, PCXGV, GXGVP, XGVPG, GVPGX ou des combinaisons de ceux-ci, où V est la valine, P est la proline, G est la glycine et X est n'importe quel acide aminé à l'exception de la proline ;
un agent de chimiosensibilisation ; et
un agent anticancéreux,
le fragment hydrophobe et/ou l'ELP conjugué au fragment hydrophobe étant encapsulé dans la bicouche lipidique.

2. Liposome selon la revendication 1, l'agent de chimiosensibilisation étant contenu dans l'espace intérieur du liposome, ou dans la bicouche lipidique.

3. Liposome selon la revendication 2, l'agent anticancéreux étant contenu dans l'espace intérieur du liposome.

4. Liposome selon la revendication 1, l'agent de chimiosensibilisation étant choisi parmi :
(a) le groupe constitué d'inhibiteurs de la protéine-1 de la résistance multiple aux médicaments (MDR1), d'inhibiteurs de la MDR-2, d'inhibiteurs de la protéine-1 liée à la résistance multiple aux médicaments (MRP-1), d'inhibiteurs de la protéine de résistance du cancer du sein (BCRP) et de combinaisons de ceux-ci ; et/ou
(b) le groupe constitué de la cyclosporine A, du vérapamil, du bricodar, du réversan et de combinaisons de ceux-ci.

5. Liposome selon l'une quelconque des revendications 1 à 4, l'agent anticancéreux étant un agent anticancéreux à base d'anthracycline.

6. Liposome selon l'une quelconque des revendications 1 à 5, comprenant en outre un agent stabilisant la bicouche lipidique.

7. Liposome selon la revendication 6, l'agent de chimiosensibilisation et/ou l'agent anticancéreux ayant une activité d'agent stabilisant la bicouche lipidique.

8. Liposome selon la revendication 6, l'agent stabilisant la bicouche lipidique comprenant un stéroïde ou un dérivé de celui-ci.

9. Liposome selon l'une quelconque des revendications 1 à 8, les motifs répétés se répétant 2 à 200 fois.

10. Liposome selon l'une quelconque des revendications 1 à 9, la bicouche lipidique comprenant des dérivés obtenus par dérivatisation avec des polymères hydrophiles.

11. Liposome selon l'une quelconque de la revendication 10, le lipide dérivé étant un dérivé de phospholipide et/ou le polymère hydrophile étant choisi parmi le polyéthylène glycol (PEG), l'acide polylactique, l'acide polyglycolique, des copolymères d'acide polylactique et d'acide polyglycolique, des alcools polyvinyliques, la polyvinylpyrrolidone, un oligosaccharide et des mélanges de ceux-ci.

12. Liposome selon l'une quelconque des revendications 1 à 11, le liposome ayant une température de transition de 39 °C à 45 °C.

13. Composition pharmaceutique chargée de délivrer un agent de chimiosensibilisation et un agent anticancéreux au niveau d'un site cible d'un sujet, la composition pharmaceutique comprenant un liposome et un véhicule ou un diluant acceptable sur le plan pharmaceutique, le liposome étant le liposome selon l'une quelconque des revendications 1 à 12.

14. Liposome selon l'une quelconque des revendications 1 à 12 ou composition pharmaceutique selon la revendication 13 pour une utilisation en vue de délivrer un agent de chimiosensibilisation et un agent anticancéreux au niveau d'un site cible d'un sujet.


<110> Samsung Electronics Co., Ltd.
<120> Liposome including elastin-like polypeptide conjugated to
   moiety containing hydrophobic group, chemosensitizer and
   anticancer agent and use thereof
<130> EP84641FZTRpau
<140> not yet assigned
   <141> herewith
<150> 10-2012-0108269
   <151> 2012-09-27
<150> 10-2011-0107055
   <151> 2011-10-19
<160> 7
<170> KopatentIn 2.0
<210> 1
   <211> 5
   <212> PRT
   <213> Artificial Sequence
<220>
   <223> elastin-like polypeptide unit sequence
<220>
   <221> VARIANT
   <222> (4)
   <223> Xaa denotes amino acid other than proline
<400> 1
   Val Pro Gly Xaa Gly
    1               5
<210> 2
   <211> 5
   <212> PRT
   <213> Artificial Sequence
<220>
   <223> elastin-like polypeptide unit sequence
<220>
   <221> VARIANT
   <222> (3)
   <223> Xaa denotes amino acid other than proline
<400> 2
   Pro Gly Xaa Gly Val
   1               5
<210> 3
   <211> 5
   <212> PRT
   <213> Artificial Sequence
<220>
   <223> elastin-like polypeptide unit sequence
<220>
   <221> VARIANT
   <222> (2)
   <223> Xaa denotes amino acid other than proline
<400> 3
   Gly Xaa Gly Val Pro
   1               5
<210> 4
   <211> 5
   <212> PRT
   <213> Artificial Sequence
<220>
   <223> elastin-like polypeptide unit sequence
<220>
   <221> VARIANT
   <222> (1)
   <223> Xaa denotes amino acid other than proline
<400> 4
   Xaa Gly Val Pro Gly
   1               5
<210> 5
   <211> 5
   <212> PRT
   <213> Artificial Sequence
<220>
   <223> elastin-like polypeptide unit sequence
<220>
   <221> VARIANT
   <222> (5)
   <223> Xaa denotes amino acid other than proline
<400> 5
   Gly Val Pro Gly Xaa
   1 5
<210> 6
   <211> 30
   <212> PRT
   <213> Artificial Sequence
<220>
   <223> elastin-like polypeptide unit sequence modified with stearoylation and amidation
<220>
   <221> VARIANT
   <222> (1)
   <223> Amino terminal nitrogen is stearoylated
<220>
   <221> VARIANT
   <222> (30)
   <223> Carboxy terminal carboxy group is amidated with -NH2
<400> 6
   Val Pro Gly Val Gly Val Pro Gly Val Gly Val Pro Gly Val Gly Val
   1 5 10 15
   Pro Gly Val Gly Val Pro Gly Val Gly Val Pro Gly Val Gly
   20 25 30
<210> 7
   <211> 15
   <212> PRT
   <213> Artificial Sequence
<220>
   <223> elastin-like polypeptide unit sequence modified with stearoylation at amino terminal and amidation at carboxy terminal
<220>
   <221> VARIANT
   <222> (1)
   <223> Amino terminal nitrogen is stearoylated
<220>
   <221> VARIANT
   <222> (15)
   <223> Carboxy terminal carboxyl group is amidated with -NH2
<400> 7
   Val Pro Gly Val Gly Val Pro Gly Val Gly Val Pro Gly Val Gly
   1 5 10 15